(19) Europäisches Patentamt
European Patent Office
Office européen des brevets

(11) Veröffentlichungsnummer: **0 270 107 B1**

(12) **EUROPÄISCHE PATENTSCHRIFT**

(45) Veröffentlichungstag der Patentschrift: **05.08.92**

(51) Int. Cl.5: **C07D 233/94**

(21) Anmeldenummer: **87117869.5**

(22) Anmeldetag: **03.12.87**

(54) **Verfahren zur Herstellung von 4(5)-Nitroimidazol-5(4)-carbonsäuren.**

(30) Priorität: **04.12.86 DE 3641514**

(43) Veröffentlichungstag der Anmeldung:
**08.06.88 Patentblatt 88/23**

(45) Bekanntmachung des Hinweises auf die Patenterteilung:
**05.08.92 Patentblatt 92/32**

(84) Benannte Vertragsstaaten:
**BE CH DE FR GB IT LI**

(56) Entgegenhaltungen:

**R. G. Fargher, F. L. Pyman; J. Chem. Soc. 115, (1919), S. 217-236**

**CHEMICAL ABSTRACTS, Band 82, Nr.11,17.März 1975, Columbus, Ohio, USA GIREVA, R.N.; ALESHINA, G.A.; REZNICHENKO, L.A.; KOCHERGIN, P.M. "Synthesis and study of some imidazole derivatives. VII. Esters and amides of nitroimidazole carboxylic acids." Seite 432, Spalte 1, Zusammenfassung-Nr. 72 869f**

**CHEMICAL ABSTRACTS, Band 51, Nr. 22, 25. November 1957, Columbus, Ohio, US; KULEV, L.P.; GIREVA, R.N. "Preparation of 4(5)-nitroimidazole-5(4)-carboxylic acid from technical 4(5)-(hydroxymethyl) imidazole." Spalte 17 897, Zusammenfassung-Nr. 17 897a**

**R.C. ELDERFIELD "Heterocyclic Compounds" Band 5, "Five-Membered Heterocycles Containing Two Hetero Atoms and Their Benzo Derivates" 1957 JOHN WILEY & SONS, INC., London - Seite 208, Zeilen 6-10**

(73) Patentinhaber: **BASF Aktiengesellschaft**
**Carl-Bosch-Strasse 38**
**W-6700 Ludwigshafen(DE)**

(72) Erfinder: **Dockner, Toni, Dr.**
**Grossgasse 6**
**W-6701 Meckenheim(DE)**
Erfinder: **Kempe, Uwe, Dr.**
**Danziger Strasse 9**
**W-6701 Dannstadt-Schauernheim(DE)**
Erfinder: **Koehler, Hermann, Dr.**
**Roentgenstrasse 7**
**W-6711 Beindersheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft ein Verfahren zur Herstellung von 4(5)-Nitroimidazol-5(4)-carbonsäuren.

Während Imidazole nach bekannten Verfahren, z.B. wie in der DE-OS 26 45 172 beschrieben, in Schwefelsäure nitriert werden können, wenn man höhere als die für die Nitrierung von carbocyclischen Aromaten üblichen Temperaturen anwendet, ist eine entsprechende Umsetzung von Imidazol-(4(5)-carbonsäuren in der Literatur nicht beschrieben. Von verschiedenen Autoren wird eine solche Umsetzung sogar als undurchführbar angesehen, so z.B. von K. Hoffmann in "The Chemistry of Heterocyclic Compounds" "Imidazole and Its Derivates", part I, 1953, Interscience Publishers, Seite 128, Absatz 3. Auch R.G. Fargher und F.L. Pyman stellen in J. Chem. Soc. 115, 217-236, insbesondere Seite 220 (1919) fest, daß die Nitrierung von Imidazolen nicht möglich ist, wenn am Imidazolring eine Carboxylgruppe vorhanden ist.

Zur Herstellung von 4(5)-Nitroimidazol-5(4)-carbonsäuren sind daher verschiedene aufwendige und umständliche Methoden gewählt worden, von denen die von C.P. Kulev und R.N. Gireva in Appl. Chem. USSR 30, 858 (1957) und CA 51, 17897a, 1957 beschriebene am elegantesten erscheint. Danach wird 4-Hydroximethylimidazol zunächst mit rauchender $HNO_3$ in 98 %iger $H_2SO_4$ zunächst bei niedriger Temperatur nitriert und dann bei höherer Temperatur die Hydroximethylgruppe oxidiert. Hachteilig an dem Verfahren ist jedoch die umständliche und in schlechter Ausbeute verlaufende Synthese des Ausgangsstoffes, ausgehend von Fructose.

Der Erfindung lag daher die Aufgabe zugrunde, einen einfachen Zugang zu 4(5)-Nitroimidazol-5(4)-carbonsäuren zu ermöglichen, nach dem die Verbindungen in guten Ausbeuten erhalten werden können.

Demgemäß wurde ein Verfahren zur Herstellung von 4(5)-Nitroimidazol-5(4)-carbonsäuren der allgemeinen Formel I

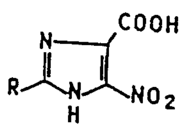 (I),

in der R Wasserstoff, $C_1$- bis $C_{20}$-Alkyl oder $C_4$- bis $C_8$-Cycloalkyl bedeutet, gefunden, welches dadurch gekennzeichnet ist, daß man Imidazol-4(5)-carbonsäuren der allgemeinen Formel II

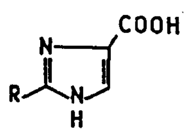 (II),

in der R die obengenannten Bedeutungen hat, bei Temperaturen von 80 bis 200 °C mit einem Gemisch aus Schwefelsäure und Salpetersäure umsetzt, wobei die Summe aller Einsatzstoffe einen Wassergehalt von nicht mehr als 5 Gew.-% enthalten.

Die Umsetzung verläuft gemäß folgender Reaktionsgleichung:

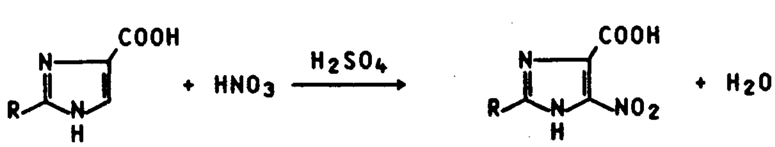


II I

R = H, aliphatischer, cycloaliphatischer, araliphatischer oder aromatischer Rest

Als Ausgangsstoffe Können in 2-Stellung unsubstituierte oder durch einen aliphatischen, cycloaliphatischen, araliphatischen oder aromatischen Rest substituierte Imidazol-4(5)carbonsäuren verwendet werden. Aliphatische Reste sind z.B. Alkylreste mit 1 bis 20, insbesondere 1 bis 10, vorzugsweise 1 bis 6 Kohlenstoffatomen. Beispielsweise sind der Methyl-, Ethyl-, n-Propyl-, Isopropyl-, n-Butyl-, sek.-Butyl- oder tert.-Butylreste

aufgeführt. Cycloaliphatische Reste sind z.B. $C_4$-$C_8$-Cycloalkylreste, z.B. Cyclohexyl oder Cyclopentyl. Araliphatische Reste sind z.B. Aralkyl- oder Alkarylreste, insbesondere mit 7 bis 12 Kohlenstoffatomen. z.B. Benzyl. 2-Phenylethyl, 3-Phenylpropyl oder 3-Phenylbutyl. Vorteilhaft trägt der Phenylrest einen desaktivierenden Substituenten. Arylgruppen sind insbesondere Phenyl oder substituiertes Phenyl, das als Substituenten vorteilhaft desaktivierende Gruppen enthält, z.B. Nitrogruppen, so daß eine Nitrierung des Aromaten vermieden wird. Die genannten Reste Können noch unter den Reaktionsbedingungen inerte Substituenten, z.B. $C_1$-$C_4$-Alkylgruppen, Cyano-, Halogen oder Nitrogruppen tragen. Die Ausgangsstoffe sind z.B. nach dem in der DE-OS 34 27 136 beschriebenen Verfahren gut zugänglich.

Die Nitrierung der Imidazol- bzw. 2-substituierten Imidazolcarbonsäuren erfolgt mit Salpetersäure im Gemisch mit Schwefelsäure, in der Regel hochkonzentrierter oder rauchender Schwefelsäure (Oleum). Im allgemeinen verwendet man eine Salpetersäure von der Dichte zwischen 1,38 bis 1,52 (20 ° C) und eine Schwefelsäure von der Dichte zwischen 1,82 und 2,0 (20 ° C).

Besonders vorteilhaft kann man Salpetersäure mit einer Konzentration von 80 bis 100 % verwenden.

Zweckmäßig wählt man in dem Gemisch von Salpetersäure und Schwefelsäure, der Nitriersäure, ein Verhältnis von 0,1 bis 1 mol Salpetersäure je Mol Schwefelsäure. In der Regel verwendet man 1 bis 4 mol, vorzugsweise 1,5 bis 2,5 mol Salpetersäure je Mol Imidazolcarbonsäure II.

Der Wassergehalt des Umsetzungsgemisches soll vor Beginn der Reaktion nicht höher als 5 Gew.-%, bezogen auf das Umsetzungsgemisch, liegen. Im allgemeinen liegt der Wassergehalt bei 0 bis 2 , insbesondere 0 bis 1 Gew.-%, bezogen auf das Umsetzungsgemisch. Das bei der Nitrierung entstehende Reaktionswasser kann vorteilhaft durch Zugabe von $SO_3$ (Oleum) gebunden werden.

Die Reaktionstemperatur beträgt mindestens 80 ° C. Sie kann oberhalb dieser Temperatur in weiten Grenzen variiert werden, in der Regel sind jedoch Temperaturen von 80 bis 200 ° C ausreichend. Vorzugsweise können Temperaturen von 80 bis 180 ° C, insbesondere 100 bis 160 ° C gewählt werden.

Die Umsetzung kann kontinuierlich oder diskontinuierlich nach den dafür üblichen Techniken, in der Regel bei Atmosphärendruck, vorgenommen werden.

Nach erfolgter Umsetzung, im allgemeinen nach 2 bis 6 Stunden bei diskontinuierlicher Fahrweise, können die Produkte in an sich bekannter Weise isoliert werden, indem man z.B. das Umsetzungsgemisch mit Eis oder Wasser verdünnt, im Gemisch die Säure mit Alkali, vorzugsweise Ammoniak, auf pH 2 bis 5 abstumpft und den sich dabei abscheidenden Endstoff abfiltriert, wäscht und trocknet. Die so erhaltenen 4-(5)-Nitro-imidazol-5(4)-carbonsäuren können durch Umfällen aus Wasser weiter gereinigt werden.

Die nach dem erfindungsgemäßen Verfahren erhaltenen Produkte stellen wertvolle Zwischenprodukte für die Synthese von Wirkstoffen, insbesondere Pharmaka und Pflanzenschutzmitteln, dar.

Beispiel 1:

Herstellung von 4(5)-Nitro-imidazol-(4)-carbonsäure

In 553 g (1,66 mol) 24 %iges Oleum wurden 112 g (1 mol) Imidazol-4(5)-carbonsäure unter Rühren langsam eingetragen und bei 100 ° C 156 g (2 mol) 81 %ige $HNO_3$ binnen 45 Minuten hinzugetropft. Das Reaktionsgemisch wurde noch 5 Stunden bei 100 ° C gerührt, abgekühlt und auf 500 g Eis gegossen. Daraufhin wurde mit Ammoniakwasser auf pH 3 gestellt und der ausgefallene Niederschlag abgesaugt, gewaschen und getrocknet. Man erhält 163 g rohe 4(5)-Nitro-imidazol-5(4)-carbonsäure mit einem Gehalt von 67,5 %, entsprechend einer Ausbeute von 70 %, bezogen auf eingesetzte Imidazol-4(5)-carbonsäure.

Das Rohprodukt wurde bei pH 11,5 in wäßriger NaOH gelöst, dann wurde mit konzentrierter Salzsäure auf pH 10 eingestellt. Der ausgefallene Feststoff wurde abfiltriert und verworfen. Daraufhin wurde bei pH 8 die Nitrocarbonsäure ausgefällt, abfiltriert, gewaschen und getrocknet. Man erhält so die reine 4(5)-Nitro-imidazol-5(4)-carbonsäure, Smp. 301 bis 303 ° C, Gehalt > 97 % (bestimmt durch Hochdruckflüssigkeitschromatographie).

Beispiel 2:

Herstellung von 2-Methyl-4(5)-nitro-imidazol-5(4)-carbonsäure

Analog Beispiel 1 wurden in 276,5 g (0,83mol) 24 %igem Oleum 63 g (0,5 mol) 2-Methylimidazol-4(5)-carbonsäure mit 78g (1 mol) 81 %iger $HNO_3$ umgesetzt und das Reaktionsgemisch wie oben beschrieben aufgearbeitet. Rohausbeute an 2-Methyl-4(5)-nitro-imidazol-5(4)-carbonsäure: 60 %.

Das Rohprodukt wurde wie beschrieben aus Wasser umgefällt. Man erhält so die reine 2-Methyl-4(5)-nitro-imidazol-5(4)-carbonsäure, Smp. 250 bis 252 ° C, Gehalt: > 99 % (bestimmt durch Hochdruckflässigkeitschromatographie).

**Patentansprüche**

1. Verfahren zur Herstellung von 4(5)-Nitro-imidazol-5(4)-carbonsäuren der allgemeinen Formel I

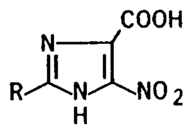

(I),

in der R Wasserstoff, $C_1$- bis $C_{20}$-Alkyl oder $C_4$- bis $C_8$-Cycloalkyl bedeutet, dadurch gekennzeichnet, daß man Imidazol-4(5)-carbonsäuren der allgemeinen Formel II

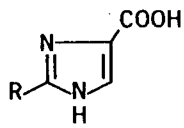

(II),

in der R die obengenannten Bedeutungen hat, bei Temperaturen von 80 bis 200 ° C mit einem Gemisch aus Schwefelsäure und Salpetersäure umsetzt, wobei die Summe aller Einsatzstoffe einen Wassergehalt von nicht mehr als 5 Gew.-% enthalten.

2. Verfahren nach Anspruch 1, dadurch gekennzeichnet, daß die Summe aller Einsatzstoffe einen Wassergehalt von nicht mehr als 1 Gew.-% enthalten.

3. Verfahren nach den Ansprüchen 1 und 2, dadurch gekennzeichnet, daß man 1 bis 4 mol Salpetersäure je Mol Imidazol-4(5)-carbonsäure verwendet.

4. Verfahren nach den Ansprüchen 1 bis 3, dadurch gekennzeichnet, daß man 0,1 bis 1 mol Salpetersäure je Mol Schwefelsäure verwendet.

5. Verfahren nach den Ansprüchen 1 bis 4, dadurch gekennzeichnet, daß man 80 bis 100 %ige Salpetersäure verwendet.

6. Verfahren nach den Ansprüchen 1 bis 5, dadurch gekennzeichnet, daß man die Umsetzung bei Temperaturen von 100 bis 160 ° C vornimmt.

**Claims**

1. A process for the preparation of a 4(5)-nitroimidazole-5(4)-carboxylic acid of the general formula I

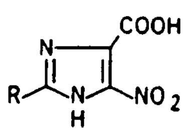

(I)

where R is hydrogen, $C_1$- to $C_{20}$-alkyl or $C_4$- to $C_8$-cycloalkyl, which comprises reacting an imidazole-4-(5)-carboxylic acid of the general formula II

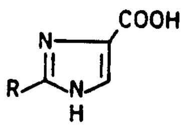

(II)

where R has the abovementioned meanings, with a mixture of sulfuric acid and nitric acid at from 80 to 200 ° C, the total water content of all starting materials being no more than 5% by weight.

**2.** A process as claimed in claim 1, wherein the total water content of all starting materials is no more than 1% by weight.

**3.** A process as claimed in claim 1 or 2, wherein from 1 to 4 moles of nitric acid are used per mole of imidazole-4(5)-carboxylic acid.

**4.** A process as claimed in any of claims 1 to 3, wherein 0.1-1 mole of nitric acid is used per mole of sulfuric acid.

**5.** A process as claimed in any of claims 1 to 4, wherein from 80 to 100% strength nitric acid is used.

**6.** A process as claimed in any of claims 1 to 5, wherein the reaction is carried out at from 100 to 160 ° C.

**Revendications**

**1.** Procédé de préparation d'acides 4(5)-nitroimidazole-5(4)-carboxyliques de formule générale I

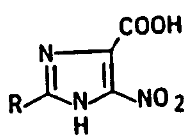

(I),

dans laquelle R représente un atome d'hydrogène, un reste alkyle en $C_1$ à $C_{20}$ ou cycloalkyle en $C_4$ à $C_8$, caractérisé en ce qu'on fait réagir des acides imidazole-4(5)-carboxyliques de formule générale II

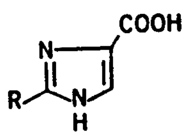

(II),

dans laquelle R a les significations susmentionnées, à des températures de 80 à 200 ° C, avec un mélange d'acide sulfurique et d'acide nitrique, la somme de tous les corps mis en réaction comprenant une teneur en eau qui ne dépasse pas 5% en poids.

**2.** Procédé selon la revendication 1, caractérisé en ce que la somme de tous les corps mis en réaction comprend une teneur en eau qui ne dépasse pas 1% en poids.

**3.** Procédé selon la revendication 1 ou 2, caractérisé en ce qu'on utilise 1 à 4 moles d'acide nitrique par mole d'acide imidazole-4(5)-carboxylique.

**4.** Procédé selon l'une quelconque des revendications 1 à 3, caractérisé en ce qu'on utilise 0,1 à 1 mole d'acide nitrique par mole d'acide sulfurique.

**5.** Procédé selon l'une quelconque des revendications 1 à 4, caractérisé en ce qu'on utilise de l'acide nitrique à 80-100%.

6. Procédé selon l'une quelconque des revendications 1 à 5, caractérisé en ce qu'on procède à la réaction à des températures de 100 à 160 °C.